(19) Europäisches Patentamt / European Patent Office / Office européen des brevets

(11) EP 3 628 280 A1

(12) DEMANDE DE BREVET EUROPEEN

(43) Date de publication:
01.04.2020 Bulletin 2020/14

(51) Int Cl.:
*A61F 7/00* (2006.01)

(21) Numéro de dépôt: 19199328.6

(22) Date de dépôt: 24.09.2019

(84) Etats contractants désignés:
AL AT BE BG CH CY CZ DE DK EE ES FI FR GB
GR HR HU IE IS IT LI LT LU LV MC MK MT NL NO
PL PT RO RS SE SI SK SM TR
Etats d'extension désignés:
BA ME
Etats de validation désignés:
KH MA MD TN

(30) Priorité: 27.09.2018 FR 1858905

(71) Demandeur: Aurore Concept
77186 Noisiel (FR)

(72) Inventeurs:
• BOUZIGON, Romain
25320 VORGES LES PINS (FR)
• QUENIART, Samuel
77186 NOISIEL (FR)

(74) Mandataire: Decorchemont, Audrey Véronique
Christèle et al
CABINET BOETTCHER
16, rue Médéric
75017 Paris (FR)

(54) **DISPOSITIF DE CRYOTHERAPIE**

(57) Dispositif de cryothérapie (1) comprenant :
- une chambre de cryothérapie (2) pour recevoir un sujet à traiter (3) ;
- un générateur de froid (4) agencé pour générer une température interne à la chambre de cryothérapie (2) inférieure d'au moins une dizaine de degrés Celsius à une température externe à la chambre de cryothérapie.

Le dispositif comporte :
- une unité centrale (5) ;
- un premier capteur (6) de mesure fonctionnellement relié à ladite unité centrale (5) pour mesurer une température cutanée d'une partie de sujet (3) se trouvant dans ladite chambre de cryothérapie (2) ; et
- un dispositif d'information (7) fonctionnellement relié à ladite unité centrale (5) pour générer un signal à l'attention du sujet (3), ladite unité centrale (5) commandant l'émission du signal (S) à l'attention du sujet en fonction de mesures de températures cutanées générées par le premier capteur de mesure (6).

Fig. 1

EP 3 628 280 A1

**Description**

La présente invention concerne le domaine des dispositifs de cryothérapie et des méthodes d'utilisation de tels dispositifs.

ARRIERE PLAN DE L'INVENTION

La cryothérapie est une méthode utilisée pour traiter des douleurs ou pour permettre une récupération physique après des efforts intensifs.

La cryothérapie est par exemple utilisée après l'entraînement des sportifs pour limiter le risque de survenue de blessures.

A cette fin, on peut placer le sujet dans une chambre de cryothérapie mise à une température interne inférieure à 0°C. Lorsqu'un temps d'exposition au froid est atteint, le praticien demande au sujet de sortir de la chambre. La séance de cryothérapie est alors terminée.

Les effets de la cryothérapie restent variables d'un sujet à l'autre et il serait souhaitable d'améliorer ces effets.

OBJET DE L'INVENTION

Un objet de la présente invention est de fournir un dispositif de cryothérapie permettant d'améliorer l'efficacité de la séance de cryothérapie,

RESUME DE L'INVENTION

A cette fin, il est proposé selon l'invention, un dispositif de cryothérapie comprenant :

- une chambre de cryothérapie pour recevoir au moins une partie d'un sujet à traiter ;
- un générateur de froid agencé pour générer une température interne à la chambre de cryothérapie inférieure d'au moins une dizaine de degrés Celsius à une température externe à la chambre de cryothérapie.

Le dispositif de cryothérapie selon l'invention est essentiellement caractérisé en ce qu'il comporte :

- une unité centrale ;
- un premier capteur de mesure fonctionnellement relié à ladite unité centrale pour mesurer une température cutanée d'une partie de sujet se trouvant dans ladite chambre de cryothérapie ; et
- un dispositif d'information fonctionnellement relié à ladite unité centrale pour générer un signal à l'attention du sujet, ladite unité centrale étant agencée de manière à commander l'émission du signal à l'attention du sujet en fonction de mesures de températures cutanées générées à l'aide dudit premier capteur de mesure.

Grâce au dispositif selon l'invention, la température cutanée du sujet placé dans la chambre est prise en compte pour la génération du message à l'attention du sujet.

Ce signal peut être lumineux et/ou sonore et peut par exemple prendre la forme d'une chaîne de caractères et/ou d'un signal vocal et/ou d'un signal sonore informant le sujet d'un évènement qui dépend de la mesure de température cutanée.

Ce signal est typiquement une instruction de fin de séance de cryothérapie émise à l'attention du sujet ou une information relative au temps restant avant la fin de la séance.

Ainsi, grâce à l'invention, la séance de cryothérapie est adaptée au sujet puisque c'est l'évolution réelle de la température cutanée du sujet qui est prise comme référence pour décider de l'émission du signal.

La réaction physiologique du sujet exposé au froid est prise en compte, pendant la séance, pour influer sur le déroulement de cette séance.

Cette séance de cryothérapie est ainsi personnalisée en fonction du sujet ce qui permet une amélioration des effets de la cryothérapie.

Typiquement, le générateur de froid est agencé pour générer une température interne à la chambre de cryothérapie d'au moins 20 degrés Celsius en dessous de la température externe à la chambre de cryothérapie.

Par exemple, la température interne de la chambre sera inférieure à 0° Celsius, préférentiellement inférieure à -20°C lorsque la température externe, c'est-à-dire la température ambiante, est comprise entre 15 et 25°C.

BREVE DESCRIPTION DES DESSINS

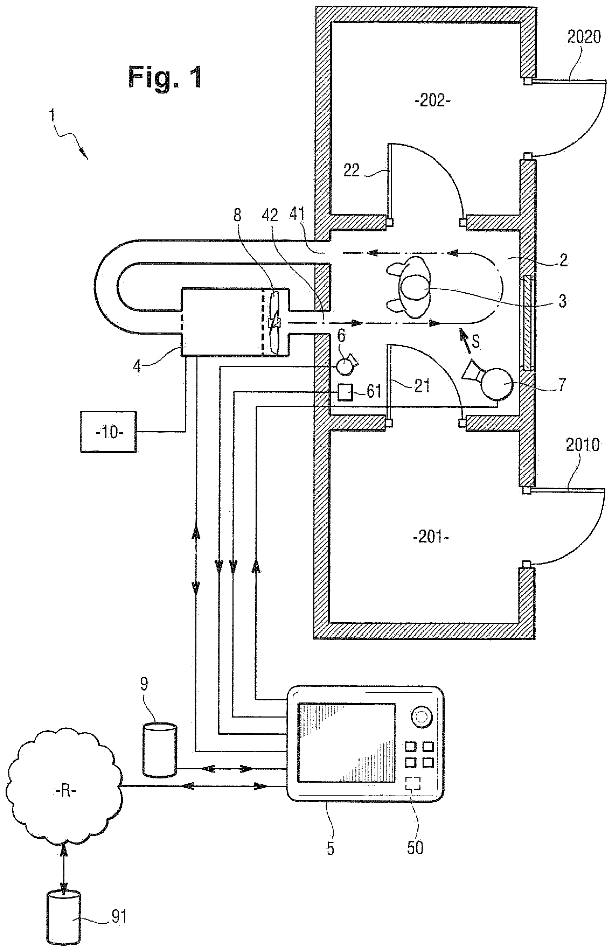

D'autres caractéristiques et avantages de l'invention ressortiront clairement de la description qui en est faite ci-après, à titre indicatif et nullement limitatif, en référence à la figure 1 annexées.

DESCRIPTION DETAILLEE DE L'INVENTION

Comme illustré à la figure 1, l'invention concerne un dispositif de cryothérapie 1 comprenant :

- une chambre de cryothérapie 2 pour recevoir au moins une partie d'un sujet à traiter 3 ;
- un générateur de froid 4 agencé pour générer une température interne à la chambre de cryothérapie 2 inférieure d'au moins 20 degrés Celsius à une température externe à la chambre de cryothérapie 2.

Préférentiellement, ce générateur de froid 4 comporte au moins une entrée d'air 41 et au moins une sortie d'air 42 qui sont reliées à la chambre de cryothérapie 2 et le dispositif de cryothérapie 1 comporte également un dispositif de soufflage d'air 8 disposé pour forcer une circulation d'air entre l'entrée d'air 41 et la sortie d'air 42 du générateur de froid 4 via le générateur de froid 4 et via la chambre de cryothérapie 2.

Ce générateur de froid 4 peut comporter un groupe froid comprenant un compresseur et un évaporateur formant échangeur thermique avec l'air forcé en circulation par le dispositif de soufflage 8.

Alternativement, le générateur de froid 4 peut comporter une réserve de gaz liquéfié reliée à un échangeur thermique pour refroidir l'air forcé en circulation par le dispositif de soufflage 8.

L'échangeur thermique du générateur de froid 4, en étant balayé par l'air forcé par le dispositif de soufflage 8 permet un ajustement de la température dans la chambre 2 et un ajustement du flux d'air froid dans la chambre.

Ces deux paramètres de fonctionnement du dispositif 1 que sont la température ambiante dans la chambre 2 et le flux d'air traversant la chambre 2 influencent directement l'échange thermique sur la peau du sujet.

Pour une meilleure répartition du froid dans la chambre 2, le générateur de froid 4 peut comporter plusieurs évaporateurs répartis à plusieurs niveaux de hauteur par rapport au niveau de sol de la chambre, certains au moins de ces évaporateurs pouvant se trouver directement dans l'enceinte de la chambre 2. Ce générateur de froid 4 peut aussi comporter plusieurs sorties d'air 42 réparties à plusieurs niveaux de hauteur par rapport au niveau de sol de la chambre. Typiquement, on peut avoir au moins deux sorties d'air froid 42, l'une à environ 1.6 m par rapport au sol et l'autre à environ 1.3 m par rapport au sol.

Il est également possible qu'une grille par exemple en nid d'abeille soit disposée dans la chambre en vis-à-vis des sorties d'air 42 pour favoriser une répartition du flux d'air sur la hauteur du patient.

Le dispositif 1 comporte également :

- une unité centrale 5 pour effectuer des calculs et commander le fonctionnement du dispositif 1 ;
- un premier capteur 6 de mesure fonctionnellement relié à ladite unité centrale 5 pour mesurer une température cutanée d'au moins une partie de sujet 3 se trouvant dans ladite chambre de cryothérapie 2 ; et
- un dispositif d'information 7 fonctionnellement relié à ladite unité centrale 5 pour générer un signal à l'attention du sujet 3.

Préférentiellement, l'unité centrale 5 est fonctionnellement reliée au générateur de froid 4 pour commander la variation d'au moins un paramètre de fonctionnement du dispositif de cryothérapie sélectionné dans le groupe de paramètres de fonctionnement comprenant:

- un débit de flux d'air transitant entre ledit générateur de froid et la chambre de cryothérapie ;

- une température cible à atteindre à l'intérieur de la chambre de cryothérapie ;
- une température cible à atteindre au niveau du générateur de froid ;
- une température cutanée cible à atteindre au niveau de la peau du sujet ;
- une consigne d'évolution temporelle de température à l'intérieur de la chambre de cryothérapie ;
- une consigne d'évolution temporelle de température cutanée à atteindre au niveau de la peau du sujet ;
- une durée prédéterminée à atteindre de séance de cryothérapie.

Comme on le voit sur la figure 1, le dispositif comporte aussi au moins une base de données 9 fonctionnement reliée à l'unité centrale 5 pour permettre à l'unité centrale, d'une part de lire dans la base de données 9 des informations stockées relatives à un sujet donné et d'autre part d'écrire des informations relatives à ce sujet donné comme des informations obtenues lors d'une séance de cryothérapie pratiquée par ce sujet.

Cette base de données 9 peut être implantée localement vis-à-vis de la chambre de cryothérapie ou être implanté à distance de la chambre de cryothérapie et être accessible via un réseau de communication R tel qu'internet.

Il est également possible que le dispositif 1 comporte plusieurs bases de données, l'une étant locale 9 et l'autre étant distante 91.

L'usage d'une base de données distante 91 peut être utile pour le cas où le dispositif de cryothérapie comporterait plusieurs chambres disposées en des endroits éloignés les uns des autres, la base de données distante 91 servant d'une part à recueillir des informations relatives de l'ensemble des sujets utilisant ces chambres et d'autre part à diffuser ces informations vers l'une des implantations où le sujet donné souhaite suivre une séance.

Préférentiellement, le dispositif 1 comporte des moyens de l'identification du sujet et des moyens de stockage d'informations personnelles de ce sujet dans la et/ou les bases de données 9, 91.

Ces moyens d'identification peuvent comporter un lecteur de badge / carte d'identification du sujet et/ou des moyens de reconnaissance optique du sujet et/ou un identifiant du sujet associé à un moyen de signature du sujet.

On peut ainsi conserver les informations de plusieurs sujets dans la / les bases de données 9, 91 et chaque sujet donné peut retrouver ses informations ou les enrichir depuis l'une quelconque des chambres de cryothérapie de la pluralité de chambres de cryothérapie.

Le dispositif de cryothérapie selon l'invention peut aussi comporter une interface homme / machine reliée à l'unité centrale pour diffuser le message à l'attention du sujet et/ou permettre de collecter et/ou visualiser des informations relatives à la séance et/ou au sujet.

Dans l'exemple illustré sur la figure 1, l'interface homme / machine comporte un écran et un clavier relié fonctionnellement à l'unité centrale 5.

Préférentiellement, l'unité centrale 5 est adaptée à commander la variation dudit au moins un des paramètres de fonctionnement du dispositif de cryothérapie en fonction d'informations relatives au sujet donné qui sont stockées dans la base de données 9.

Les informations relatives à un sujet donné sont ici représentatives du morphotype du sujet, comme son poids, sa taille, son âge, son sexe, son indice de masse corporelle, son état de santé.

Ces informations peuvent aussi comprendre le nombre de séances de cryothérapie déjà suivies par ce sujet, un historique de caractéristiques physiologiques du sujet mesurées pendant ces séances de cryothérapie.

Préférentiellement, le dispositif de cryothérapie 1 selon l'invention comporte une source autonome d'alimentation en énergie 10 (par exemple un groupe électrogène) pour alimenter en énergie le générateur de froid 4, l'unité centrale 5, le premier capteur de mesure 6 et le dispositif d'information 7.

Le fonctionnement autonome du dispositif de cryothérapie 1 facilite son déplacement pour par exemple l'utiliser dans le cadre d'évènements sportifs.

Il est possible que le dispositif de cryothérapie 1 selon l'invention comporte aussi des roues pour faciliter son déplacement. Par exemple il peut être mobile à la manière d'une remorque ou d'un camion.

Comme indiqué précédemment, l'unité centrale 5 est agencée de manière à commander l'émission du signal S à l'attention du sujet se trouvant dans la chambre 2, au moins en fonction de mesures de températures cutanées générées à l'aide dudit premier capteur de mesure 6.

Comme on le verra par la suite, la génération de ce même signal S peut aussi être commandée en fonction d'autres mesures ou critères prédéterminés.

Ce signal S est délivré via le dispositif d'information 7 qui peut comporter un haut-parleur et/ou un éclairage pour visualiser le signal S.

Ce dispositif d'information 7 peut être uniquement disposé dans la chambre 2 pour informer prioritairement le patient et éventuellement un opérateur situé hors de la chambre 2.

Alternativement, ce dispositif d'information 7 peut être uniquement disposé hors de la chambre pour informer l'opérateur et le patient.

Enfin, ce dispositif d'information 7 peut être réparti entre l'intérieur et l'extérieur de la chambre pour ajuster la forme du signal S émise à l'intérieur de la chambre 2 pour le sujet et ajuster la forme du signal S émise à l'extérieur de la chambre pour l'opérateur.

L'ajustement de forme du signal S, peut consister à ajuster la nature du signal S qui peut être sonore ou visuel, le niveau de puissance de ce signal, le contenu du signal (message vocal ou message textuel ou simple stimulation par sons / lumières).

Préférentiellement, l'unité centrale 5 est reliée fonctionnellement au dispositif de soufflage 8 pour commander une variation de vitesse de circulation d'air entre l'entrée d'air 41 et la sortie d'air 42 en fonction de mesures de températures cutanées générées à l'aide dudit premier capteur de mesure 6.

Préférentiellement, l'unité centrale 5 est aussi reliée fonctionnellement au générateur de froid pour commander sa température propre et ainsi influer sur la température ambiante dans la chambre 2.

En permettant le contrôle de ces paramètres de température ambiante dans la chambre et de flux d'air traversant la chambre, on peut affiner l'effet de la séance sur le sujet et ainsi contribuer à la personnalisation de la séance en fonction de l'objectif et des réactions du sujet.

Il est également possible d'améliorer la personnalisation de la séance en faisant en sorte que l'unité centrale fasse varier les paramètres de température ambiante dans la chambre 2 et de flux d'air en fonction de la mesure de température cutanée du sujet.

Grâce à l'invention, le déroulement de la séance de cryothérapie dépend de l'évolution de la température cutanée du sujet pendant la séance.

On améliore ainsi la personnalisation de la séance tout en limitant les risques pour le sujet.

Le premier capteur de mesure 6 est agencé pour mesurer une température cutanée par mesure de rayonnements infrarouges provenant de la peau de ladite partie de sujet 3 se trouvant dans ladite chambre de cryothérapie 2. Préférentiellement, on fait en sorte que ce premier capteur de mesure 6 permette au moins de mesurer une température cutanée au niveau de l'abdomen du sujet. A cette fin, ce premier capteur de mesure 6 est au moins agencé pour mesurer une température cutanée dans une zone située de part et d'autre d'un niveau de hauteur à 1,2 m par rapport au sol de la chambre 2.

Typiquement, ce premier capteur de mesure 6 comporte une tête de détection de rayonnements infrarouges disposée dans la chambre de cryothérapie pour y détecter des rayonnements infrarouges provenant du sujet.

Préférentiellement cette tête génère une image infrarouge de la partie du sujet se trouvant dans la chambre de cryothérapie. La température cutanée mesurée peut être une température cutanée maximale courante ou une température cutanée minimale courante ou une moyenne de plusieurs températures cutanées courantes.

Un tel capteur 6 peut par exemple être un capteur de la société Optris de type CT LT.

Alternativement, ce capteur pourrait être un capteur de température mis en contact contre la peau du sujet.

Préférentiellement, la chambre de cryothérapie 2 est adaptée à accueillir l'intégralité du sujet 3 dans la chambre 2, cette chambre comportant au moins une porte 21 pour l'accès du sujet dans la chambre. Ainsi le dispositif permet de réaliser une cryothérapie corps entier.

Préférentiellement, la chambre de cryothérapie 2 est placée le long d'au moins une pièce adjacente qui peut être reliée audit générateur de froid pour former une pièce tampon refroidie pour servir d'accès à la chambre de cryothérapie 2. Le sujet peut ainsi passer par une pièce refroidie pendant une trentaine de seconde avant de pénétrer dans la chambre de cryothérapie. La pièce adjacente et la chambre de cryothérapie peuvent avoir des températures et/ou taux d'humidité différents et ajustés par l'unité centrale 5. Cette configuration favorise une évaporation cutanée dans la pièce adjacente, avant que le sujet ne pénètre dans la chambre de cryothérapie 2. Ceci favorise l'homéostasie du sujet.

Dans le mode de réalisation illustré, la chambre de cryothérapie 2 est placée entre deux pièces adjacentes 201, 202 et elle est séparée de chacune de ces pièces via une porte 21, 22.

Chacune de ces pièces adjacentes 201, 202 comporte une porte propre 2010, 2020 donnant vers l'extérieur du dispositif. Ces pièces adjacentes 201, 202 forment des sas pour l'accès et la sortie de la chambre de cryothérapie. Comme indiqué précédemment, les températures et taux d'humidité respectifs de la chambre et des pièces adjacentes peuvent être ajustés indépendamment par l'unité centrale 5 et le générateur de froid 4, en fonction des besoins du sujet.

On limite ainsi les pertes d'énergie lors de l'ouverture ou de la fermeture de la chambre de cryothérapie puisque celle-ci n'est accessible que via des sas fermés 201, 202.

Eventuellement, ces sas peuvent être utilisés comme pièce de change pour les sujets.

La chambre de cryothérapie est préférentiellement équipée d'une fenêtre 23 pour permettre à un opérateur et au sujet d'échanger visuellement.

Dans le mode de la figure 1, le dispositif 1 comporte un deuxième capteur de mesure 61 fonctionnellement relié à ladite unité centrale 5 pour mesurer au moins une caractéristique physiologique du sujet se trouvant dans ladite chambre de cryothérapie 2, cette au moins une caractéristique physiologique appartenant à un groupe de caractéristiques physiologiques comprenant un rythme cardiaque du sujet, une pression sanguine du sujet, fréquence respiratoire, un volume respiratoire, une saturation en oxygène du sang du sujet (par exemple le deuxième capteur de mesure 61 peut être un oxymètre de pouls ou saturomètre).

L'unité centrale 5 est ici agencée de manière à commander l'émission du signal S à l'attention du sujet en fonction de mesures de ladite au moins une caractéristique physiologique mesurée à l'aide dudit deuxième capteur de mesure 61.

Ce deuxième capteur de mesure 61 peut être constitué par un ensemble de plusieurs cellules de détection / mesure disposées de manière à mesurer plusieurs desdites caractéristiques physiologiques du sujet présent dans la chambre de cryothérapie.

Par exemple, on peut ainsi décider de délivrer un signal S de fin de séance si l'on détecte un comportement anormal du sujet.

L'unité centrale peut ainsi générer le signal S si au moins une condition prédéterminée parmi plusieurs conditions est vérifiée. De telles conditions prédéterminées sont par exemple l'atteinte d'un écart de température cutanée, d'une température cible cutanée, d'une limite de rythme cardiaque, de ventilation ou d'oxygénation attendu ou atteinte d'une durée prédéterminée maximale de séance de cryothérapie.

Préférentiellement, l'unité centrale 5 est agencée pour enregistrer dans la base de données 9 des caractéristiques physiologiques du sujet 3 mesurées par ce deuxième capteur 61 lors d'une séance de cryothérapie.

Nous allons maintenant décrire la manière dont le dispositif selon l'invention peut être paramétré pour que les conditions de la séance de cryothérapie à réaliser soient personnalisées en fonction du sujet à traiter.

A cette fin, l'unité centrale 5 est préférentiellement adaptée à commander la variation dudit au moins un paramètre de fonctionnement du dispositif de cryothérapie 1 en fonction d'une valeur d'objectif sélectionnée dans un groupe de valeurs d'objectifs prédéterminé comprenant une première valeur correspondant à un objectif de traitement de la douleur, une seconde valeur correspondant à un objectif de récupération physique après un effort, une troisième valeur correspondant à un objectif de bien être, ces première, seconde et troisième valeurs étant différentes les unes des autres.

En sélectionnant l'une de ces valeurs parmi ces première, seconde et troisième valeurs distinctes, l'unité centrale 5 induit une variation dudit au moins un des paramètres de fonctionnement du dispositif de cryothérapie qui est spécifique à cette valeur sélectionnée.

Ainsi, en fonction du morphotype du sujet obtenu via les informations relatives au sujet stockées dans la base de données 9 et en fonction de l'objectif de la séance de cryothérapie obtenu via la valeur sélectionnée, l'unité centrale 5 détermine le ou les paramètres de fonctionnement du dispositif de cryothérapie pour la séance à venir.

La séance est ainsi personnalisée en fonction du sujet à traiter et en fonction de l'objectif de la séance.

L'unité centrale 5 est agencée pour commander la variation dudit au moins un paramètre de fonctionnement du dispositif de cryothérapie en fonction de mesures de la température cutanée du sujet obtenues à l'aide du premier capteur de mesure 6.

Ainsi, pendant la séance on peut faire varier le ou les paramètres de fonctionnement du dispositif 1 en fonction de l'évolution de la température cutanée mesurée du sujet.

Ceci permet de prendre en compte l'évolution de paramètres physiologiques du sujet mesurés avec ce premier capteur 6 et/ou à l'aide du second capteur 61 pour déterminer l'évolution des paramètres de fonctionnement du dispositif.

La séance de cryothérapie est ainsi adaptable en fonction :

- des réactions du sujet mesurées pendant la séance ;
- de l'objectif de la séance (à l'aide de la valeur représentative de l'objectif de la séance) ; et
- des paramètres morphologiques du sujet (âge, sexe, indice de masse corporelle...) .

La commande de variation de ces paramètres de fonctionnement du dispositif de cryothérapie, résulte de l'exécution par l'unité centrale exécute d'un logiciel de supervision. Ce logiciel peut être diffusé via le réseau R pour une mise à jour de différentes unités 5 reliées à ce réseau.

Le dispositif selon l'invention selon l'invention permet de réaliser une cryothérapie corps entier tout en variant des paramètres de la séance de cryothérapie en fonction de mesures de températures cutanées effectuées à l'intérieur de la chambre 2.

Selon un premier mode de réalisation, on peut faire en sorte que l'unité centrale 5 commande l'émission du signal S à l'attention du sujet lorsqu'une température cutanée courante mesurée à l'aide dudit premier capteur de mesure 6 est égale à une température cutanée cible prédéterminée. Comme on le verra par la suite la détermination d'une telle température cible permet de personnaliser la séance de cryothérapie en fonction de l'état du sujet et de son objectif.

Selon un autre exemple, on peut faire en sorte que l'unité centrale 5 commande l'émission du signal S à l'attention du sujet lorsqu'une valeur prédéterminée de baisse de température cutanée a été détectée. L'unité centrale 5 est ainsi agencée pour détecter l'atteinte de ladite valeur prédéterminée de baisse de température en calculant un écart entre une valeur de température cutanée mesurée de début de séance et une valeur de température cutanée courante, ces valeurs de températures cutanée étant générées à l'aide dudit premier capteur de mesure 6.

Selon un autre aspect, l'invention concerne également un procédé d'utilisation du dispositif de cryothérapie selon l'invention, dans lequel le dispositif de cryothérapie 1 comporte un calculateur 50 agencé pour calculer une température cutanée cible devant être atteinte en fin de séance.

L'unité centrale est agencée de manière à commander l'émission du signal S à l'attention du sujet lorsque cette température cutanée cible est atteinte (la température cutanée est estimée via des mesures de températures réalisées à l'aide dudit premier capteur de mesure 6).

Le calculateur 50 étant agencé pour calculer cette température cutanée cible à l'aide d'un coefficient représentatif de l'âge du sujet C1, d'un coefficient représentatif de l'objectif de la séance Tn, d'un coefficient représentatif de l'indice de masse corporelle du sujet C2 et d'un coefficient représentatif du sexe du sujet C3.

Ainsi, la température cible à atteindre est calculée de manière personnalisée avant le début de la séance, en prenant en compte différentes informations relatives au sujet.

De manière préférentielle, la température cutanée cible Tx en °C est donnée par la formule :

$$Tx = Tn * C1 * C2 * C3$$

dans laquelle

- Tn est une température de besoin choisie entre 10 et 15°C en fonction de l'objectif de la séance. Par exemple Tn est égale à 10°C si l'objectif de la séance est de traiter des douleurs sévères, Tn est égale à 12°C si l'objectif de la séance est une récupération après un exercice physique, Tn est égale à 14°C si l'objectif de la séance est de fournir un bien-être et Tn est égale à 15°C si l'objectif de la séance est de traiter des douleurs modérées.
- C1 est un coefficient représentatif de l'âge du sujet. Par exemple, C1 prend une valeur de 1.0 si le sujet a moins de 55 ans et une valeur 1.2 si le sujet a plus de 55 ans.
- C2 est un coefficient représentatif de l'indice de masse corporelle du sujet IMC. L'indice de masse corporelle est calculé en divisant la masse du sujet exprimée en kilogrammes par le carré de la taille du sujet exprimée en mètre.

Par exemple, C2 prend :

- une valeur de 1.0 si l'indice de masse corporel du sujet est considéré comme normal et compris entre 18.5 et 30 ;
- une valeur de 1.1 si l'indice de masse corporelle du sujet est inférieur à 18.5 ; et
- une valeur de 0.9 si l'indice de masse corporelle du sujet est supérieur à 30.

- C3 est un coefficient représentatif du sexe du sujet. C3 prend une valeur de 1.2 si le sujet est une femme et une valeur 1.0 si le sujet si le sujet est un homme.

Avec cette formule de calcul, on constate que plus l'organisme du sujet est susceptible de réagir rapidement au froid auquel il est exposé (par exemple parce que le sujet présente peu de graisses ou qu'il est jeune ou qu'il s'agit d'un homme) et plus la température cutanée cible Tx sera élevée.

A contrario, plus l'organisme du sujet est susceptible de réagir lentement au froid (par exemple parce que le sujet présente beaucoup de graisses ou qu'il est âgé ou qu'il s'agit d'une femme) et plus la température cutanée cible Tx sera abaissée.

Par exemple, pour une séance de récupération physique (Tx=12°C) pour un sujet homme (C3= 1.0) de 50 ans (C1=1.0) et d'indice de masse corporelle de 25 (C2=1.0), on aura Tx = 12 * 1.0 * 1.0 *1.0 = 12°C.

Le signal S délivré au sujet destiné à signaler la fin de séance de cryothérapie sera émis lorsque la température cutanée cible Tx de 12°C sera mesurée par le premier capteur 6.

La mesure en temps réel de la température cutanée associée à l'adaptation de la température cible Tx en fonction de la morphologie et de l'âge du sujet permet d'obtenir des effets améliorés pour le sujet.

## Revendications

1. Dispositif de cryothérapie (1) comprenant :

   - une chambre de cryothérapie (2) pour recevoir au moins une partie d'un sujet à traiter (3) ;
   - un générateur de froid (4) agencé pour générer une température interne à la chambre de cryothérapie (2) inférieure d'au moins une dizaine de degrés Celsius à une température externe à la chambre de cryothérapie,

   **caractérisé en ce qu'**il comporte :

   - une unité centrale (5) ;
   - un premier capteur (6) de mesure fonctionnellement relié à ladite unité centrale (5) pour mesurer une température cutanée d'une partie de sujet (3) se trouvant dans ladite chambre de cryothérapie (2) ; et
   - un dispositif d'information (7) fonctionnellement relié à ladite unité centrale (5) pour générer un signal à l'attention du sujet (3), ladite unité centrale (5) étant agencée de manière à commander l'émission du signal (S) à l'attention du sujet en fonction de mesures de températures cutanées générées à l'aide dudit premier capteur de mesure (6).

2. Dispositif de cryothérapie selon la revendication 1, dans lequel l'unité centrale (5) est agencée de manière à commander l'émission du signal (S) à l'attention du sujet lorsqu'une valeur prédéterminée de baisse de température cutanée a été détectée, l'unité centrale (5) étant agencée pour détecter l'atteinte de ladite valeur prédéterminée de baisse de température en calculant un écart entre une valeur de température cutanée mesurée de début de séance et une valeur de température cutanée courante, ces valeurs de températures cutanée étant générées à l'aide dudit premier capteur de mesure (6).

3. Dispositif de cryothérapie selon l'une quelconque des revendications 1 ou 2, dans lequel le générateur de froid (4) comporte une entrée d'air (41) et une sortie d'air (42) qui sont reliées à la chambre de cryothérapie (2) et le dispositif de cryothérapie (1) comporte également un dispositif de soufflage d'air (8) disposé pour forcer une circulation d'air entre l'entrée d'air (41) et la sortie d'air (42) du générateur de froid (4) via le générateur de froid (4) et via la chambre de cryothérapie (2).

4. Dispositif de cryothérapie selon la revendication 3, dans lequel l'unité centrale (5) est reliée fonctionnellement au dispositif de soufflage (8) pour commander une variation de vitesse de circulation d'air entre l'entrée d'air (41) et la sortie d'air (42) en fonction de mesures de températures cutanées générées à l'aide dudit premier capteur de mesure (6).

5. Dispositif de cryothérapie selon l'une quelconque des revendications 1 à 4, dans lequel le premier capteur de mesure (6) est agencé pour mesurer une température cutanée par mesure de rayonnements infrarouges provenant de la peau de ladite partie de sujet (3) se trouvant dans ladite chambre de cryothérapie (2).

6. Dispositif de cryothérapie selon l'une quelconque des revendications 1 à 5, dans lequel la chambre de cryothérapie est adaptée à accueillir l'intégralité du sujet (3) dans la chambre (2), cette chambre comportant au moins une porte (21) pour l'accès du sujet dans la chambre.

7. Dispositif de cryothérapie selon l'une quelconque des revendications 1 à 6, comportant en outre au moins une base de données (9) fonctionnement reliée à l'unité centrale (5) pour permettre à l'unité centrale, d'une part de lire dans la base de données (9) des informations stockées relatives à un sujet donné et d'autre part d'écrire des informations obtenues lors d'une séance de cryothérapie pratiquée par ce sujet donné.

8. Dispositif de cryothérapie selon la revendication 7, dans lequel l'unité centrale (5) est fonctionnellement reliée au générateur de froid pour commander la variation d'au moins un paramètre de fonctionnement du dispositif de cryothérapie sélectionné dans le groupe de paramètres de fonctionnement comprenant :

   - un débit de flux d'air transitant entre ledit générateur de froid et la chambre de cryothérapie ;
   - une température cible à atteindre à l'intérieur de la chambre de cryothérapie ;
   - une température cible à atteindre au niveau du générateur de froid ;
   - une température cutanée cible à atteindre au niveau de la peau du sujet ;
   - une consigne d'évolution temporelle de température à l'intérieur de la chambre de cryothérapie ;

- une consigne d'évolution temporelle de température cutanée à atteindre au niveau de la peau du sujet ;

- une durée prédéterminée à atteindre de séance de cryothérapie.

9. Dispositif de cryothérapie selon la revendication 8, dans lequel l'unité centrale (5) est adaptée à commander la variation dudit au moins un des paramètres de fonctionnement du dispositif de cryothérapie en fonction desdites informations relatives à un sujet donné qui sont stockées dans ladite base de données (9).

10. Dispositif de cryothérapie selon la revendication 9, dans lequel l'unité centrale (5) est adaptée à commander la variation dudit au moins un paramètre de fonctionnement du dispositif de cryothérapie (1) en fonction d'une valeur d'objectif sélectionnée dans un groupe de valeurs d'objectifs prédéterminé comprenant une première valeur correspondant à un objectif de traitement de la douleur, une seconde valeur correspondant à un objectif de récupération physique après un effort, une troisième valeur correspondant à un objectif de bien être, ces première, seconde et troisième valeurs étant différentes les unes des autres.

11. Dispositif de cryothérapie selon l'une quelconque des revendications 1 à 10, comportant :

- un deuxième capteur de mesure fonctionnellement (61) relié à ladite unité centrale (5) pour mesurer au moins une caractéristique physiologique du sujet se trouvant dans ladite chambre de cryothérapie (2), cette au moins une caractéristique physiologique appartenant à un groupe de caractéristiques physiologiques comprenant un rythme cardiaque du sujet, une pression sanguine du sujet, fréquence respiratoire, un volume respiratoire, une saturation en oxygène du sang du sujet ; et

- ladite unité centrale (5) étant agencée de manière à commander l'émission du signal (S) à l'attention du sujet en fonction de mesures de ladite au moins une caractéristique physiologique mesurée à l'aide dudit deuxième capteur de mesure.

12. Dispositif de cryothérapie selon la revendication 11, dans lequel ladite unité centrale (5) est agencée pour enregistrer dans la base de données (9) des caractéristiques physiologiques du sujet (3) mesurées par le deuxième capteur (61) lors d'une séance de cryothérapie.

13. Dispositif de cryothérapie selon l'une quelconque des revendications 1 à 12, dans lequel l'unité centrale (5) est agencée pour que le signal (S) à l'attention du sujet soit également émis en cas d'atteinte d'une durée prédéterminée maximale de séance de cryothérapie.

14. Dispositif de cryothérapie selon l'une quelconque des revendications 1 à 13, comportant une source autonome d'alimentation en énergie (10) pour alimenter en énergie le générateur de froid (4), l'unité centrale (5), le premier capteur de mesure (6) et le dispositif d'information (7).

15. Procédé d'utilisation du dispositif de cryothérapie selon l'une quelconque des revendications 1 à 14, dans lequel le dispositif de cryothérapie comporte un calculateur (50) agencé pour calculer une température cutanée cible devant être atteinte en fin de séance, ladite unité centrale (5) étant agencée de manière à commander l'émission du signal (S) à l'attention du sujet lorsque cette température cutanée cible est atteinte (via les mesures de températures cutanées générées à l'aide dudit premier capteur de mesure), le calculateur (50) étant agencé pour calculer cette température cutanée cible à l'aide d'un coefficient représentatif de l'âge du sujet (C1), d'un coefficient représentatif de l'objectif de la séance (Tn), d'un coefficient représentatif de l'indice de masse corporelle du sujet (C2) et d'un coefficient représentatif du sexe du sujet (C3).

**Fig. 1**

Europäisches
Patentamt

European
Patent Office

Office européen
des brevets

# RAPPORT DE RECHERCHE EUROPEENNE

Numéro de la demande

EP 19 19 9328

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2014/148706 A1 (VAN TREECK CHRISTOPH [DE] ET AL) 29 mai 2014 (2014-05-29) | 1-10,14 | INV. A61F7/00 |
| Y | * alinéas [0030] - [0042]; figure 1 * * alinéas [0057] - [0062]; figure 3 * ----- | 11,12 | |
| X | EP 3 195 834 A1 (CRYOTECH NORDIC OÜ [EE]) 26 juillet 2017 (2017-07-26) * alinéas [0051] - [0056]; figure 5 * ----- | 1-6,13, 14 | |
| Y | WO 2017/205775 A1 (GREENE BRANDON [US]; GREENE CRYSTAL [US]) 30 novembre 2017 (2017-11-30) * alinéas [0123] - [0129]; figures 1A-B, 5A-6B * * alinéas [0169] - [0172]; figure 18 * ----- | 11,12 | |
| A | US 2018/000352 A1 (STARR KAREN [US] ET AL) 4 janvier 2018 (2018-01-04) * alinéas [0081] - [0096] * * alinéas [0109] - [0118]; figures 9-10 * ----- | 1-14 | |

DOMAINES TECHNIQUES RECHERCHES (IPC)

A61F

Le présent rapport a été établi pour toutes les revendications

| Lieu de la recherche | Date d'achèvement de la recherche | Examinateur |
|---|---|---|
| La Haye | 10 février 2020 | Schnurbusch, Daniel |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 19 19 9328

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

10-02-2020

| Document brevet cité au rapport de recherche | | Date de publication | Membre(s) de la famille de brevet(s) | | Date de publication |
|---|---|---|---|---|---|
| US 2014148706 | A1 | 29-05-2014 | DE 102011077522 | A1 | 20-12-2012 |
| | | | EP 2720607 | A2 | 23-04-2014 |
| | | | ES 2559625 | T3 | 15-02-2016 |
| | | | US 2014148706 | A1 | 29-05-2014 |
| | | | WO 2012172026 | A2 | 20-12-2012 |
| EP 3195834 | A1 | 26-07-2017 | DK 3195834 | T3 | 25-03-2019 |
| | | | EP 3195834 | A1 | 26-07-2017 |
| | | | ES 2713254 | T3 | 20-05-2019 |
| | | | FI 126899 | B | 31-07-2017 |
| | | | PL 3195834 | T3 | 28-06-2019 |
| | | | US 2017209302 | A1 | 27-07-2017 |
| WO 2017205775 | A1 | 30-11-2017 | US 2017340477 | A1 | 30-11-2017 |
| | | | WO 2017205775 | A1 | 30-11-2017 |
| US 2018000352 | A1 | 04-01-2018 | AUCUN | | |

EPO FORM P0460

Pour tout renseignement concernant cette annexe : voir Journal Officiel de l'Office européen des brevets, No.12/82